# EUROPEAN PATENT SPECIFICATION

(11) **EP 2 699 173 B1**
(45) Date of publication and mention of the grant of the patent: **03.05.2017**
(21) Application number: 12773692.4
(22) Date of filing: 18.04.2012
(51) Int. Cl.: A61B 17/28, A61B 17/062

(54) **SURGICAL NEEDLE HOLDER**
CHIRURGISCHER NADELHALTER
PORTE-AUIGUILLE CHIRURGICAL

(30) Priority: 18.04.2011 SE 1150339
(43) Date of publication of application: 26.02.2014
(73) Proprietor: Frimand Rönnow, Carl-Fredrik, 211 43 Malmö (SE)
(72) Inventor: Frimand Rönnow, Carl-Fredrik, 211 43 Malmö (SE)
(74) Representative: Awapatent AB
(86) International application number: PCT/SE2012/050416
(87) International publication number: WO 2012/144950

(56) References cited:
- EP-A1- 1 421 910
- DE-A1- 2 025 868
- DE-C- 273 689
- DE-U1- 20 220 841
- GB-A- 2 280 397
- GB-A- 190 228 711
- US-A- 3 577 991
- US-A- 3 823 719
- US-A- 5 876 420
- US-B1- 7 758 609

## Description

### Field of the invention

The present invention relates to a surgical device comprising a first leg and a second leg pivotally connected at a fulcrum arranged between a first part and a second part of each leg; an engagement device arranged at the first part of each leg for engagement with an object, such as tissue or a surgical needle; a finger grip in the form of a substantially closed loop arranged at the second part of each leg; the legs being arranged in a plane which is perpendicular to a symmetry plane through the fulcrum, whereby the legs are arranged at each side of the symmetry plane in a substantially mirror arrangement, the second part of each leg extending from the fulcrum in a first portion having an increased distance to the symmetry plane; and in a second portion comprising the finger grips.

### Background of the invention

Within the medical field, it is very common to use different types of surgical devices such as forceps, both generally within medicine and more specifically within surgery. Two common types of forceps are needle holders, which are used for holding surgical needles, and hemostatic forceps used for the closing of arteries.

When suturing, i.e. closing an opening in a body part of a patient, the user, such as a surgeon, uses a needle holder for holding a surgical needle. One common type of needle holder is the so called Mayo-Hegar, also known as Ryder or French Eye. This type of needle holder has a design which is very similar to that of a common pair of scissors. But instead of sharp edges for cutting at the front of the "scissors", the Mayo-Hegar needle holder is equipped with jaws for holding the surgical needle firmly. Also, the "scissors" are equipped with a locking member which locks the needle holder when holding the needle. The needle holder is released by separating the legs of the needle holder sideways, i.e. in a direction which is perpendicular to that of the plane of the needle holder.

The Mayo-Hegar needle holder has some drawbacks. Firstly, the needle might not release easily from the needle holder, hence causing tissue damage when the needle holder is being removed. Secondly, and more importantly, holding the needle holder at the intended location, i.e. at the finger grips of the needle holder, does not allow for a more refined suturing action with higher demands on precision. On the contrary, the most precision and control is achieved when holding the needle holder at the front end of the needle holder, i.e. closer to the above mentioned jaws, and when holding the needle holder entirely on the outside instead of locking ones fingers in the finger grips which reduces ones flexibility,. One also achieves better strength when using this type of front end grip, which is advantageous e.g. when performing surgery on thicker tissue. Further, when using this grip, it is also possible to access with the needle at a 90° angle against the tissue in order to cause the least damage. When doing this with the Mayo-Hegar needle holder one repeatedly needs to change the grip when locking or releasing the needle. Except for being time-consuming, this change of grip may also cause traction in the tissue leading to an inferior result. In addition, the Mayo-Hegar needle holder may not give satisfactory stability in hand, for surgical precision. These drawbacks are all important, especially within plastic surgery.

Another type of needle holder is the so called Mathieu. The Mathieu needle holder has two legs with a spring therein between, which provides an outwards directed force. Instead of being provided with finger grips, the legs are connected, at the end opposite the end which is provided with jaws, by a locking member, hence counteracting the outwards directed force. The Mathieu needle holder is suitable when needing to perform the above discussed refined suturing action with higher precision, control, and strength. However, the hand of the user is always very close to the area that is being sutured which is a drawback when suturing deeper tissues, in angles, or in inaccessible areas.

A needle holder of the Mayo-Hegar type is disclosed in the publication US 5,662,665 A.

U.S. Patent No. 3,823,719 discloses a hand-operated clamp type surgical instrument comprising two cooperating arms pivotably interconnected along a single axis, each of said arms comprising a jaw member located at one end of said arm and a finger loop located at the other end of said arm. Said instrument includes a ratchet locking device and has the ability to eliminate excessive pressure at the jaws due to the presence of a heel and a shoulder stop in said ratchet locking device.

DE 20 25 868 discloses a clamping or holding forceps comprising a latch which automatically exerts its action during pressing together of the forceps shanks due to the pivotal arrangement of one of the latching parts.

DE 202 20 841 discloses a surgical instrument provided with at least two branches which can be moved to one another or relative to one another for actuating a tool at a distal end of the instrument, wherein the branches each carry a holding ring projecting in a proximal region of the branches.

U.S. Patent No. 5,876,420 discloses a needle holder comprising finger grips and a locking device arranged to be engaged by movement of finger grips towards each other and to be released by movement of the finger grips further in a direction towards each other.

### Summary of the invention

It is an object of the present invention to mitigate the above problems, and to provide a surgical device which facilitates both holding at the front end of the surgical device and holding at the finger grips of the surgical device, and where one does not have to change the grip when locking or releasing an object being held by the surgical device. The invention provides a surgical device according to claim 1. Further embodiments of the invention are provided in the dependent claims. These objects are achieved by a surgical device comprising a first leg and a second leg pivotally connected at a fulcrum arranged between a first part and a second part of each leg; an engagement device arranged at the first part of each leg for engagement with a surgical needle; a finger grip in the form of a substantially closed loop arranged at the second part of each leg; the legs being arranged in a plane which is perpendicular to a symmetry plane through the fulcrum, whereby the legs are arranged at each side of the symmetry plane in a substantially mirror arrangement, the second part of each leg extending from the fulcrum in a first portion having an increased distance to the symmetry plane; and in a second portion comprising the finger grips; wherein the finger grip is connected to the second portion of each leg at a connection point in which a centre of the finger grip is positioned closer to the symmetry plane than the connection point.

The use of finger grips connected to each leg at a connection point where the centre of each finger grip is positioned closer to the symmetry plane than the connection point provides a surgical device which can be used comfortably both by holding at the finger grips of the surgical device and by holding at the front end of the surgical device, and which supports the users grip when suturing with holding at the front end. Since the surgical device has two intended types of grips, the user does not need to neither change instruments, e.g. between the two previously discussed types of surgical devices, nor use the surgical device in a way which is not intended. Further, the user may hold the surgical device using all fingers, which facilitates a very stable surgical device since the surgical device is in direct contact with all fingers and much of the palm. Also, it is possible to both lock and release the object being held by the surgical device when holding at the front end of the surgical device, which means that one does not need to change grips due to this. Hence, one both saves time and avoids damaging tissue.

In one embodiment, the second portion of each leg extends along an outside of the finger grip in relation to the symmetry plane. The second portion of each leg may also form a convex curvature in the area of the finger grip, whereby the finger grip is positioned inside the convex curvature. These embodiments further facilitate the possibility of both locking and releasing the needle when holding at the front end of the needle holder. The surgical device further comprises a spring device for urging the legs in a direction away from each other. The spring device provides an outwardly directed force for opening the surgical device, i.e. separating the legs of the surgical device, which force counteracts a closing force, e.g. that of the hand of the user. The spring device also makes it possible to both lock and release the object being held by the surgical device when holding at the front end of the surgical device.

The legs are releasably locked to each other by a locking device. This locking device locks the surgical device such that the user does not need to tension his/her grip around the surgical device and hence the object being held by the surgical device.

The locking device is arranged to be engaged by movement of the finger grips towards each other and which is arranged to be released by movement of the finger grips further in a direction towards each other. This type of locking device further facilitates both locking and releasing the needle when holding at the front end of the needle holder.

The engagement device is a jaw device arranged at the first part of each leg, the jaw device being arranged to hold and engage with a surgical needle.

The area between the finger grips may be free from leg portions, which is a way of assuring a more versatile use since it is easier for the user to find the most comfortable and suitable individual grip when there are no obstructions between the finger grips of the needle holder. Further, tissue or fingers do not get stuck between the finger grips as easily.

### Brief description of the drawings

This and other aspects of the present invention will now be described in more detail, with reference to the appended drawings showing currently preferred embodiments of the invention.
Figure 1 shows an elevational view of an embodiment of the needle holder according to the present invention.

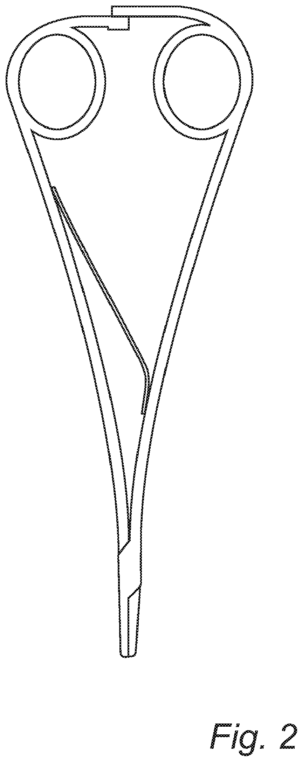

Figure 2 shows an elevational view of another embodiment of a needle holder according to the present invention.
Figure 3 shows an elevational view of a further embodiment of a needle holder according to the present invention.
Figure 4 shows an elevational view of another embodiment of a needle holder according to the present invention.
Figure 5 shows a perspective view of yet another embodiment of the needle holder according to the present invention.

### Detailed description

The invention will be described below with reference to a needle holder. The inventive concept can, however, be applied to other types of surgical devices, such as hemostatic forceps.

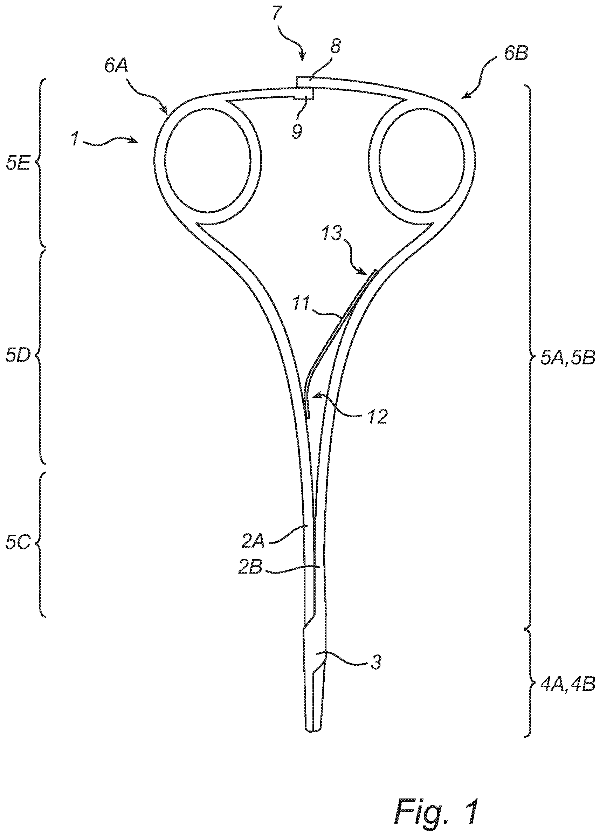

Figure 1 shows a needle holder 1 for holding surgical needles. The needle holder 1 has a basic design which is similar to that of a pair of scissors or pliers. The needle holder 1 comprises two legs 2a, 2b each having a first part 4a, 4b and a second part 5a, 5b. The legs are pivotably connected by a fulcrum 3, such as a pin extending perpendicular to the plane of the needle holder, intermediate the first part 4a, 4b and the second part 5a, 5b of the legs 2a, 2b. The legs 2a, 2b generally extend at each side of a symmetry plane parallel with the pin 3 so that the legs 2a and 2b are substantially mirror shapes of each other in the symmetry plane. The plane of the needle holder is perpendicular to the symmetry plane.

The first part 4a, 4b of the legs 2a, 2b comprises engagement members such as jaws for holding a surgical needle. The second part 5a, 5b of the legs 2a, 2b comprises finger grips 6a, 6b. Each finger grip has an oval loop shape with a centre point at the centre of each oval.

In the embodiment shown in Fig. 1, the second part 5a, 5b of each leg comprises a first portion having an increasing distance to the symmetry plane, and a second portion comprising the finger grips. The first portion, in turn, comprises a first section 5c which is essentially parallel with the symmetry plane and a second section 5d which is concave and extends away from the symmetry plane. The second portion comprises a third section 5e which is convex. Such a design makes the needle holder 1 as slim as possible so that it takes up only a small space, and would also prevent tissue from being squeezed or getting caught in the spacing between the legs 2a, 2b.

The second part 5a, 5b of each leg 2a, 2b comprises said finger grip 6a, 6b. Each finger grip 6a, 6b extends from the corresponding leg portion towards the symmetry plane. Each finger grip 6a, 6b is arranged at a predetermined distance from the fulcrum 3, i.e. at a distance which is appropriate for a specific size of needle holder 1. Needle holders 1 usually come in a number of predetermined sizes, according to standards within the industry.

Each finger grip 6a, 6b is arranged so that the centre of the oval loop of the finger grip is positioned closer to the symmetry plane than the corresponding leg portion. With this arrangement, it is easier to press the legs 2a, 2b towards each other in order to release the needle holder. The locking device and the procedure for releasing the locking device are described further below.

The second portion of each leg 2a, 2b extends along the outside of the finger grip in relation to the symmetry plane. By that is meant that the second portion of the legs 2a, 2b form a soft curvature which is not only comfortable to hold but which provides a stable handle when the needle holder 1 is held with the hand surrounding the outside of the second portion of the legs of the needle holder 1. A soft, smooth curvature means that there are no sharp angles or bends, such that the curvature suits the inside of the hand and can be held by all of the users fingers, and such that the needle holder 1 can be held comfortably regardless of the placement of the hand, should it be at the front end or at the finger grips. Hence, the finger grip 6a, 6b is connected to the second portion of each leg 2a, 2b with the centre of the finger grip closer to the symmetry plane than the connection point. It is also preferable that the outside of the legs 2 is grooved or knurled in order to improve the grip further, e.g. by helping to prevent the needle holder from slipping out of the users' hand.

The legs 2 preferably have a width which increases in the area from the fulcrum 3 towards the finger grips 6a, 6b, i.e. in sections 5c and 5d. The width could be of any suitable dimensions, but is preferably between 3 and 12 mm. By width is meant the thickness of each leg as seen from the narrowest side of the needle holder, i.e. in a direction which is perpendicular to the plane of the needle holder. A larger width will provide a more stable needle holder, when holding at the front end of the needle holder.

Further, the end of the second part 5a, 5b of the legs 2a, 2b, i.e. the third section 5e, comprises a locking device 7. I.e., the second part 5a, 5b of each leg 2a, 2b extends such that it passes the finger grips 6a, 6b. Each leg 2a, 2b is thereafter bent towards the opposing leg 2b, 2a. The very ends 8, 9 of the legs 2a, 2b are then engaged such that they releasably lock the second parts 5a, 5b of the two legs 2a, 2b to each other. The second part 5a, 5b of the legs 2a, 2b consequently forms a closed loop between the fulcrum 3 and the locking device 7.

The locking device 7 comprises a hook 8 and corresponding grooves 9, where the hook 8 is arranged on one leg 2a and the grooves 9 are arranged on the other leg 2b such that the hook and grooves engage in certain positions. When the hook 8 and grooves 9 engage by movement of said finger grips towards each other, they lock the needle holder 1 in a desired position, i.e. a position in which the surgical needle is firmly held by the needle holder 1.

When releasing the locking device 7 by moving the finger grips 6a, 6b further in a direction towards each other i.e. releasing the hook 8 from the grooves 9, the legs 2a, 2b can be freely pivoted once again around the fulcrum 3, hence releasing the needle from the jaws.

The locking device 7 could also comprise a conventional ratchet device, i.e. which is arranged to be released by movement of the finger grips in a direction which is perpendicular to the locking movement. Such a conventional locking device, as shown in US 5,662,665 A, is however not as beneficial as the locking device described above, as regards not having to change grips when locking and releasing the needle holder.

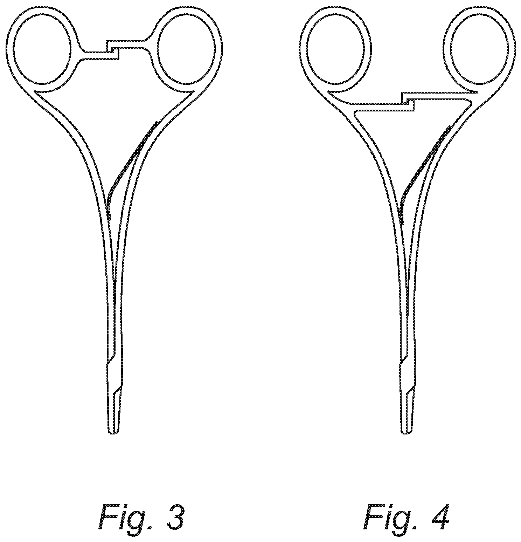

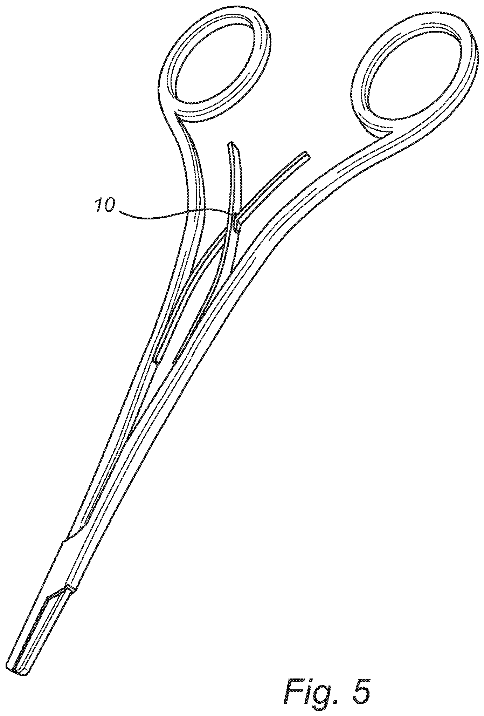

However, the locking device does not have to comprise a hook and grooves at all, it could also comprise a spring arrangement or some other type of simple locking mechanism. For example, one leg of the needle holder could be inserted into a slot in the opposing leg, such that the first leg runs inwards and outwards in the slot of the second leg. In the same way, and as shown in Figure 5, a member of a spring arrangement, located between the legs of the needle holder, could be inserted into a slot 10 in an opposing member, such that the first member runs inwards and outwards in the slot 10 of the second member. In this way, a non-locking spring action is formed. Of course, one could combine different types of locking devices as one sees fit. E.g., it is possible to combine the above mentioned hook 8 and grooves 9 with the slotted leg/spring member solution. Also, the slotted legs/ members could be locked against each other, e.g. using a small tap which hooks into the slot such as shown in Figure 5. As shown in Figures 3 and 4, the locking device 7 does not need to be arranged at the very end of the legs 2a, 2b, but could be located at any suitable place within the second part 5a, 5b of the legs 2a, 2b such as between the fulcrum 3 and the finger grips 6a, 6b, at sections 5c or 5d, or between the finger grips 6a, 6b, at section 5e. In this case, the legs 2a, 2b of the needle holder do not need to extend beyond the finger grips 6a, 6b, but could preferably end at the finger grips 6a, 6b.

Also, a spring device 11, e.g. a leaf spring, is located inside the above mentioned closed loop which is formed and extends between the fulcrum 3 and the locking device 7, i.e. the first end 12 of the spring 11 is attached to the inside of the second part 5a of one leg 2a while the other end 13 of the spring 11 is attached to the inside of the second part 5b of the opposing leg 2b. One of the ends 12 is slidably attached to the leg 2a, i.e. such that the concerned spring end 12 can slide along the inside of the leg 2a depending on the position of the legs 2a, 2b relative each other, while the other end 13 is firmly attached to the inside of the opposing leg 2b. This spring 11 could be replaced with a spring arrangement such as that shown in Figure 4. A spring device or arrangement is intended for urging the legs 2a, 2b in a direction from each other.

Further, the needle holder 1 could be equipped with other devices, such as a scissor- or cutter-like device placed at the outside of one of the legs 2, in the area of the fulcrum 3. This would assure simple cutting of the suture thread, also without changing the grip substantially.

The needle holder 1 could be manufactured of any suitable medical grade material such as metal, e.g. stainless steel, or plastics.

The person skilled in the art realizes that the present invention by no means is limited to the preferred embodiments described above. On the contrary, many modifications and variations are possible within the scope of the appended claims. For example, the locking device could have any suitable appearance. Also, the shape of the legs could be any shape which facilitates release of the needle without having to change grips. Further, the outside of the needle holder could be provided with other suitable, additional features besides, or replacing, the mentioned cutter-like device. Additionally, the loop forming the finger grip need not necessarily be completely closed, as long as any opening in the loop is small enough not to jeopardise a secure finger grip. Also, as mentioned previously, the disclosure applies not only to needle holders but all types of surgical devices, such as e.g. hemostatic forceps.

## Claims

1. Surgical device adapted for use with a first type of grip, wherein the surgical device is held at finger grips (6a, 6b), and with a second type of grip, wherein the surgical device is held at a front end, the surgical device comprising:
a first leg (2a) and a second leg (2b) pivotally connected at a fulcrum (3) arranged between a first part (4a, 4b) and a second part (5a, 5b) of each leg;
an engagement device arranged at the first part (4a, 4b) of each leg (2a, 2b) for engagement with a surgical needle; wherein said engagement device is a jaw device arranged at the first part (4a, 4b) of each leg, said jaw device being arranged to hold and engage with a surgical needle; a finger grip (6a, 6b) in the form of a substantially closed loop arranged at the second part (5a, 5b) of each leg;
said legs (2a, 2b) being arranged in a plane which is perpendicular to a symmetry plane through said fulcrum (3), whereby said legs are arranged at each side of said symmetry plane in a substantially mirror arrangement,
said second part (5a, 5b) of each leg extending from said fulcrum (3) in a first portion having an increasing distance to said symmetry plane; and in a second portion comprising said finger grips; wherein said finger grip (6a, 6b) of said second portion of said second part (5a, 5b) of each leg (2a, 2b) extends from the corresponding leg portion towards the symmetry plane at a connection point; wherein a centre of said finger grip is positioned closer to said symmetry plane than said connection point when the surgical device is in a closed position;
a locking device (7) for releasably locking the second parts (5a, 5b) of each leg (2a, 2b) to each other; wherein said locking device (7) is arranged to be engaged by movement of said finger grips (6a, 6b) towards each other and which is arranged to be released by movement of the finger grips (6a, 6b) further in a direction towards each other; wherein said locking device can be both locked and released without changing the type of grip, and
a spring device (11) for urging said legs (2a, 2b) in a direction away from each other.

2. The surgical device according to claim 1, wherein the second portion of each leg (2a, 2b) extends along an outside of said finger grip (6a, 6b) in relation to said symmetry plane.

3. The surgical device according to claim 2, wherein said second portion of each leg (2a, 2b) forms a convex curvature in the area of said finger grip (6a, 6b), whereby said finger grip is positioned inside said convex curvature.

4. The surgical device according to any one of the previous claims, wherein the area between the finger grips (6a, 6b) is free from leg portions.

## Patentansprüche

1. Chirurgische Vorrichtung, die für die Verwendung mit einer ersten Art von Griff, wobei die chirurgische Vorrichtung an Fingergriffen (6a, 6b) gehalten ist, und mit einer zweiten Art von Griff geeignet ist, wobei die chirurgische Vorrichtung an einem vorderen Ende gehalten wird, wobei die chirurgische Vorrichtung Folgendes umfasst:
einen ersten Schenkel (2a) und einen zweiten Schenkel (2b), die schwenkend an einem Drehpunkt (3) verbunden sind, der zwischen einem ersten Teil (4a, 4b) und einem zweiten Teil (5a, 5b) jedes Schenkels angeordnet ist,
eine Eingriffsvorrichtung, die am ersten Teil (4a, 4b) jedes Schenkels (2a, 2b) angeordnet ist, um eine chirurgische Nadel in Eingriff zu nehmen,
wobei die Eingriffsvorrichtung eine Backenvorrichtung ist, die am ersten Teil (4a, 4b) jedes Schenkels angeordnet ist, wobei die Backenvorrichtung zum Halten und Ineingriffnehmen einer chirurgischen Nadel angeordnet ist,
einen Fingergriff (6a, 6b) in der Form einer im Wesentlichen geschlossenen Schlaufe, der am zweiten Teil (5a, 5b) jedes Schenkels angeordnet ist,
wobei die Schenkel (2a, 2b) in einer Ebene angeordnet sind, die senkrecht zu einer Symmetrieebene durch den Drehpunkt (3) verläuft, wobei die Schenkel in einer im Wesentlichen spiegelbildlichen Anordnung an jeder Seite der Symmetrieebene angeordnet sind,
wobei sich der zweite Teil (5a, 5b) jedes Schenkels von dem Drehpunkt (3) in einem ersten Abschnitt mit einer zunehmenden Entfernung von der Symmetrieebene und in einem zweiten Abschnitt, der die Fingergriffe umfasst, erstreckt,
wobei sich der Fingergriff (6a, 6b) des zweiten Abschnitts des zweiten Teils (5a, 5b) jedes Schenkels (2a, 2b) von dem entsprechenden Schenkelabschnitt zu der Symmetrieebene an einer Verbindungsstelle erstreckt, wobei eine Mitte des Fingergriffs näher an der Symmetrieebene als die Verbindungsstelle positioniert ist, wenn die chirurgische Vorrichtung in einer geschlossenen Position ist,
eine Verriegelungsvorrichtung (7) zum lösbaren Verriegeln der zweiten Teile (5a, 5b) jedes Schenkels (2a, 2b) aneinander, wobei die Verriegelungsvorrichtung (7) so angeordnet ist, dass sie eingerückt wird, wenn die Fingergriffe (6a, 6b) aufeinander zu bewegt werden, und so angeordnet ist, dass sie gelöst wird, wenn die Fingergriffe (6a, 6b) weiter in einer Richtung aufeinander zu bewegt werden, wobei die Verriegelungsvorrichtung ohne Änderung der Griffart sowohl verriegelt als auch gelöst werden kann, und
eine Federvorrichtung (11), um die Schenkel (2a, 2b) in einer Richtung weg voneinander zu drängen.

2. Chirurgische Vorrichtung nach Anspruch 1, wobei sich der zweite Abschnitt jedes Schenkels (2a, 2b) entlang einer Außenseite des Fingergriffs (6a, 6b) in Bezug auf die Symmetrieebene erstreckt.

3. Chirurgische Vorrichtung nach Anspruch 2, wobei der zweite Abschnitt jedes Schenkels (2a, 2b) im Bereich des Fingergriffs (6a, 6b) eine konvexe Krümmung bildet, wobei der Fingergriff innerhalb der konvexen Krümmung positioniert ist.

4. Chirurgische Vorrichtung nach einem der vorhergehenden Ansprüche, wobei der Bereich zwischen den Fingergriffen (6a, 6b) frei von Schenkelabschnitten ist.

## Revendications

1. Dispositif chirurgical conçu pour une utilisation avec un premier mode de préhension, le dispositif chirurgical étant tenu au niveau d'éléments de préhension digitale (6a, 6b), et avec un second mode de préhension, le dispositif chirurgical étant tenu au niveau d'une extrémité avant, le dispositif chirurgical comprenant :
une première branche (2a) et une seconde branche (2b) raccordées de manière pivotante au niveau d'un pivot (3) disposé entre une première partie (4a, 4b) et une seconde partie (5a, 5b) de chaque branche ;
un dispositif de mise en prise disposé au niveau de la première partie (4a, 4b) de chaque branche (2a, 2b) et destiné à venir en prise avec une aiguille chirurgicale ;
ledit dispositif de mise en prise étant un dispositif à mâchoires disposé au niveau de la première partie (4a, 4b) de chaque branche, ledit dispositif à mâchoires étant conçu pour tenir une aiguille chirurgicale et venir en prise avec celle-ci ;
un élément de préhension digitale (6a, 6b) sous la forme d'une boucle essentiellement fermée disposée au niveau de la seconde partie (5a, 5b) de chaque branche ;
lesdites branches (2a, 2b) étant disposées dans un plan qui est perpendiculaire à un plan de symétrie à travers ledit pivot (3), lesdites branches étant ainsi disposées de chaque côté dudit plan de symétrie selon un agencement essentiellement spéculaire,
ladite seconde partie (5a, 5b) de chaque branche s'étendant à partir dudit pivot (3) en une première portion présentant une distance croissante vis-à-vis dudit plan de symétrie ; et en une seconde portion comprenant lesdits éléments de préhension digitale ;
ledit élément de préhension digitale (6a, 6b) de ladite seconde portion de ladite seconde partie (5a, 5b) de chaque branche (2a, 2b) s'étendant à partir de la portion de branche correspondante en direction du plan de symétrie au niveau d'un point de raccordement ; un centre dudit élément de préhension digitale étant positionné plus près dudit plan de symétrie que ledit point de raccordement lorsque le dispositif chirurgical se trouve dans une position fermée ;
un dispositif de verrouillage (7) servant à verrouiller de manière libérable les secondes parties (5a, 5b) de chaque branche (2a, 2b) de sorte qu'elles soient assujetties l'une à l'autre ; ledit dispositif de verrouillage (7) étant conçu pour être enclenché par un déplacement desdits éléments de préhension digitale (6a, 6b) en direction l'un de l'autre et étant conçu pour être libéré par un déplacement des éléments de préhension digitale (6a, 6b) plus avant dans une direction les rapprochant l'une de l'autre ; ledit dispositif de verrouillage pouvant être aussi bien verrouillé que libéré sans changer de mode de préhension, et
un dispositif formant ressort (11) servant à solliciter lesdites branches (2a, 2b) dans une direction les éloignant l'une de l'autre.

2. Dispositif chirurgical selon la revendication 1, dans lequel la seconde portion de chaque branche (2a, 2b) s'étend le long d'une partie extérieure dudit élément de préhension digitale (6a, 6b) par rapport audit plan de symétrie.

3. Dispositif chirurgical selon la revendication 2, dans lequel ladite seconde portion de chaque branche (2a, 2b) forme une courbe convexe dans la région dudit élément de préhension digitale (6a, 6b), ledit élément de préhension digitale étant ainsi positionné à l'intérieur de ladite courbe convexe.

4. Dispositif chirurgical selon l'une quelconque des revendications précédentes, dans lequel la région entre les éléments de préhension digitale (6a, 6b) est dépourvue de portions de branche.
